# EUROPEAN PATENT SPECIFICATION

(11) **EP 4 170 021 B1**
(45) Date of publication and mention of the grant of the patent: **25.09.2024**
(21) Application number: 21870556.4
(22) Date of filing: 03.12.2021
(51) Int. Cl.: C12N 5/20, C12N 15/13, C07K 16/28, G01N 33/74, G01N 33/58, G01N 33/577, C07K 16/12, G01N 33/558

(54) **MOUSE ANTI-MCR-1 PROTEIN HYBRIDOMA CELL STRAIN, MONOCLONAL ANTIBODY, AND APPLICATION**
MOUSE MCR-1 PROTEIN-RESISTENTE HYBRIDOMA-ZELLLINIE, MONOCLONALANTIBODY (MAB) UND VERWENDUNG DAVON
SOUCHE CELLULAIRE D'HYBRIDOME DE PROTÉINE ANTI-MCR-1 DE SOURIS, ANTICORPS MONOCLONAL ET APPLICATION

(30) Priority: 25.08.2021 CN 202110978479
(43) Date of publication of application: 26.04.2023
(73) Proprietor: Tianjin Era Biology Technology Co., Ltd., Tianjin 300457 (CN)
(72) Inventor: XIA, Bin, Tianjin 300480 (CN); HE, Yongsheng, Tianjin 300480 (CN); LI, Keke, Tianjin 300480 (CN); CHEN, Xiaoling, Tianjin 300480 (CN); YUAN, Qinghua, Tianjin 300480 (CN); WANG, Yamiao, Tianjin 300480 (CN); KONG, Di, Tianjin 300480 (CN); WANG, Siyi, Tianjin 300480 (CN)
(74) Representative: Bayramoglu et al.
(86) International application number: PCT/CN2021/135407
(87) International publication number: WO 2023/024313

(56) References cited:
- WO-A1-2018/158573
- WO-A1-2019/204496
- CN-A- 111 487 417
- CN-A- 113 416 710
- US-A1- 2019 322 727
- US-A1- 2019 322 727
- WANG JIAYI ET AL: "Rapid one-step enzyme immunoassay and lateral flow immunochromatographic assay for colistin in animal feed and food", JOURNAL OF ANIMAL SCIENCE AND BIOTECHNOLOGY, 17 October 2019 (2019-10-17), London, pages 1 - 10, XP055799020, Retrieved from the Internet <URL:https://jasbsci.biomedcentral.com/track/pdf/10.1186/s40104-019-0389-7.pdf> [retrieved on 20210427], DOI: 10.1186/s40104-019-0389-7
- HONGHUI WANG ET AL: "Rapid detection of colistin resistance protein MCR-1 by LC-MS/MS", CLINICAL PROTEOMICS, SPRINGER, US, vol. 16, no. 1, 26 February 2019 (2019-02-26), pages 1 - 10, XP021271268, ISSN: 1542-6416, DOI: 10.1186/S12014-019-9228-2

## Description

### TECHNICAL FIELD

The present disclosure belongs to the technical field of antibody preparation, and in particular to a mouse MCR-1 protein-resistant hybridoma cell line, and a monoclonal antibody (mAb) and use thereof.

### BACKGROUND

Immunodiagnostic reagents are the fastest-growing segment of *in vitro* diagnostic reagents, which achieve qualitative or quantitative detection through specific binding between an antigen and an antibody.

Polymyxins are currently the last choice for the clinical treatment of infections caused by multi-drug-resistant and pan-drug resistant Gram-negative bacteria. In recent years, due to the increasing clinical use of polymyxins, the number of polymyxin-resistant strains has increased year by year. Although the resistance of bacteria to polymyxins is not strong so far, the case of no cure may occur. How to effectively curb the spread of drug resistance has become a global public health issue.

Among a variety of drug resistance mechanisms, the *mcr-1* gene related to mediating the addition of lipid A phosphoethanolamine (PEtn) is discovered in various plasmids. Through the horizontal transference of a plasmid in different bacteria, the *mcr-1* gene even can coexist with other drug resistance genes in the same plasmid, and thus multiple drug resistance mechanisms are generated after expression. In addition, the unreasonable use of antibiotics makes the drug resistance of bacteria continue to increase, causing great trouble for clinicians to apply antibiotics. US 2019/322727 discloses monoclonal antibodies reacting with melanocortin-1 receptor protein (MCR-1) and their use in ELISA allowing detection of MCR-1 positive bacteria in ground meats. Detection of colistin by ELISA and LFIA is also reported in Wang Jiayi et al., "Rapid one-step enzyme immunoassay and lateral flow immunochromatographic assay for colistin in animal feed and food", Journal of animal science and biotechnology, 17 October 2019, pages 1-10. Rapid detection of colistin resistance protein MCR-1 by liquid chromatography tandem mass spectrometry LC-MS/MS is disclosed in HONGHUI WANG et al., "Rapid detection of colistin resistance protein MCR-1 by LC-MS/MS", CLINICAL PROTEOMICS, vol. 16, no. 1, 26 February 2019, pages 1-10.

Laboratory detection of *mcr-1*-mediated polymyxin resistance is mainly conducted by molecular biotechnology. The research and development of products for the rapid diagnosis of *mcr-1* with independent intellectual property rights is of great significance for the rapid identification of polymyxin-resistant strains and resistance mechanisms thereof, the optimization of polymyxin treatment regimens, and the prolongation of a service life of polymyxins as the last-line treatment drug.

### SUMMARY

In order to solve the above technical problems, the present disclosure provides a mouse MCR-1 protein-resistant hybridoma cell line, and a mAb and use thereof.

The present disclosure adopts the following technical solutions:
A mouse anti-MCR-1 protein antibody is provided, wherein the mouse anti-MCR-1 protein antibody is: an antibody 1BH8 including a light chain variable region and a heavy chain variable region, wherein the light chain variable region includes complementary-determining region (CDR) L1 shown in SEQ ID NO: 1, CDRL2 shown in SEQ ID NO: 2, and CDRL3 shown in SEQ ID NO: 3; and the heavy chain variable region includes CDRH1 shown in SEQ ID NO: 4, CDRH2 shown in SEQ ID NO: 5, and CDRH3 shown in SEQ ID NO: 6, the light chain variable region may have an amino acid sequence shown in SEQ ID NO: 7, and the heavy chain variable region may have an amino acid sequence shown in SEQ ID NO: 9;
SEQ ID NO: 1 RSSQNIVCSYGNPCLE (CDRL1)
SEQ ID NO: 2 KVSNRFS (CDRL2)
SEQ ID NO: 3 FQGSHVPWT (CDRL3)
SEQ ID NO: 4 GYTFTNYIMH (CDRH1)
SEQ ID NO: 5 YINPYNDGTKYNEKFKG (CDRH2)
SEQ ID NO: 6 GPYGNYAMDY (CDRH3)
SEQ ID NO: 7 
DVLMTQTPLSLPVSLGDQASISCRSSQNIVCSYGNPCLEWYLQKPGQSPKLLIYK
VSNRFSGVPDRFSGSGSGTDFTLKISRVEAEDLGVYYCFQGSHVPWTFGGGTELEIKR
SEQ ID NO: 9 
VQLQQSGPELVKPGASVKMSCRASGYTFTNYIMHWVKQKPGQGLDWIGYINPY
NDGTKYNEKFKGKATLSSDKSSGTAYMDLSSLTSEDSAVYYCARGPYGNYAMDYW
GQGTSVTVSSor,
an antibody 1BD9 including a light chain variable region and a heavy chain variable region, wherein the light chain variable region includes CDRL1 shown in SEQ ID NO: 11, CDRL2 shown in SEQ ID NO: 12, and CDRL3 shown in SEQ ID NO: 13; and the heavy chain variable region includes CDRH1 shown in SEQ ID NO: 14, CDRH2 shown in SEQ ID NO: 15, and CDRH3 shown in SEQ ID NO: 16, the light chain variable region may have an amino acid sequence shown in SEQ ID NO: 17, and the heavy chain variable region may have an amino acid sequence shown in SEQ ID NO: 19.
SEQ ID NO: 11 RASQDISNYLN (CDRL1)
SEQ ID NO: 12 YTSRLHS (CDRL2)
SEQ ID NO: 13 QQGDMIPDT (CDRL3)
SEQ ID NO: 14 GYAFTSCNMY (CDRH1)
SEQ ID NO: 15 YFDPYSGDAYYNQKFKD (CDRH2)
SEQ ID NO: 16 WLQLYYALDY (CDRH3)
SEQ ID NO: 17 
AIQMTQTPSSLSASLGDRVTISCRASQDISNYLNWYQQKPDGTVKLLIYYTSRLHS
GVPSRFSGSGSGTDYSLNISNLEQEDFATYFCQQGDMIPDTFGGGTKLEIKRADAAPT
SEQ ID NO: 19

VKLQQSGPELVKPGASVKVSCKASGYAFTSCNMYWVKQSHGKSLEWIGYFDPY
SGDAYYNQKFKDKATLTVDKSSSTAYMHLNSLTSEDSAVYYCASWLQLYYALDYW
GQGTSVTVSAAKTT.

A nucleic acid is provided, including nucleotides encoding the mouse anti-MCR-1 protein antibody described above.

Preferably, a nucleotide sequence of the nucleic acid for encoding the light chain variable region of the antibody 1BH8 may be as shown in SEQ ID NO: 8, and a nucleotide sequence of the nucleic acid for encoding the heavy chain variable region of the antibody 1BH8 may be as shown in SEQ ID NO: 10; and
SEQ ID NO: 8 
GATGTTTTGATGACCCAGACGCCACTCTCCCTGCCTGTCAGTCTTGGAGATCA
AGCCTCCATCTCTTGCAGATCTAGTCAGAACATTGTATGTAGTTATGGAAACCCCT
GTTTAGAATGGTACCTGCAGAAACCAGGCCAGTCTCCAAAGCTCCTGATCTACAA
AGTTTCCAACCGATTTTCTGGGGTCCCAGACAGGTTCAGTGGCAGTGGATCAGGG
ACAGATTTCACACTCAAGATCAGCAGAGTGGAGGCTGAGGATCTGGGAGTTTATT
ACTGCTTTCAAGGTTCACATGTTCCGTGGACGTTCGGTGGAGGCACCGAGCTGGA
AATCAAACGG
SEQ ID NO: 10 
GTGCAGCTTCAGCAATCAGGACCTGAGCTGGTAAAGCCTGGGGCTTCAGTGA
AGATGTCCTGCAGGGCTTCTGGATACACATTCACTAACTATATTATGCACTGGGT
GAAGCAGAAGCCTGGGCAGGGCCTTGATTGGATTGGATATATTAATCCTTACAAT
GATGGTACTAAGTACAATGAGAAGTTCAAAGGCAAGGCCACACTGTCTTCAGAC
AAATCCTCCGGCACAGCCTATATGGACCTCAGCAGCCTGACCTCTGAGGACTCTG
CGGTCTATTACTGTGCGAGAGGCCCCTATGGTAACTATGCTATGGACTACTGGGG
TCAAGGAACCTCAGTCACCGTCTCTTCA
 or,
a nucleotide sequence of the nucleic acid for encoding the light chain variable region of the antibody 1BD9 may be as shown in SEQ ID NO: 18, and a nucleotide sequence of the nucleic acid for encoding the heavy chain variable region of the antibody 1BD9 may be as shown in SEQ ID NO: 20.
SEQ ID NO: 18 
GCTATCCAGATGACACAGACTCCATCCTCCCTGTCTGCCTCTCTGGGAGACAG
AGTCACCATCAGTTGCAGGGCAAGTCAGGACATTAGCAATTATTTAAACTGGTAT
CAGCAGAAACCAGATGGAACTGTTAAACTCCTGATCTACTACACATCAAGATTAC
ACTCAGGAGTCCCATCAAGGTTCAGTGGCAGTGGGTCTGGAACAGATTATTCTCT
CAACATTAGCAACCTGGAGCAGGAAGATTTTGCCACTTACTTTTGCCAACAGGGT
GATATGATTCCGGACACGTTCGGAGGGGGGACCAAACTGGAAATAAAACGGGCT
GATGCTGCACCAACT
SEQ ID NO: 20

GTGAAGCTGCAGCAATCTGGACCTGAGCTGGTGAAGCCTGGGGCCTCAGTGA
AGGTCTCCTGCAAGGCTTCTGGTTATGCATTCACTAGCTGCAACATGTACTGGGT
GAAACAGAGCCATGGAAAGAGCCTTGAGTGGATTGGATATTTTGATCCTTACAGT
GGTGATGCTTACTACAACCAGAAGTTCAAGGACAAGGCCACATTGACTGTTGACA
AGTCCTCCAGCACAGCCTACATGCATCTCAACAGCCTGACATCTGAGGACTCTGC
AGTCTATTACTGTGCAAGTTGGTTACAACTTTACTATGCTCTAGACTACTGGGGTC
AAGGAACCTCAGTCACCGTCTCCGCAGCCAAAACGACA

A reagent comprising the mouse anti-MCR-1 protein antibody described above for detecting an MCR-1 protein antigen is provided.

Preferably, the mouse anti-MCR-1 protein antibody may be used in an *in vitro* diagnostic kit or a microfluidic chip; and the *in vitro* diagnostic kit may be a colloidal gold immunoassay kit, a chemiluminescence kit, a radioimmunoassay kit, an enzyme-linked immunosorbent assay (ELISA) kit, or a fluoroimmunoassay kit.

Preferably, an immune colloidal gold test strip of a double-antibody sandwich method may be prepared, wherein the antibody 1BH8 is used as a coating antibody and the antibody 1BD9 is used as a gold-labeled antibody; or
the antibody 1BD9 is used as a coating antibody and the antibody 1BH8 is used as a gold-labeled antibody.

The present disclosure has the following advantages and positive effects: The present disclosure provides two mouse MCR-1 protein-resistant hybridoma cell lines, which can produce two mouse anti-MCR-1 protein antibodies. According to systematic evaluation including evaluation on antibody subtype and titer, and kit sensitivity, specificity, and stability, the mouse anti-MCR-1 protein mAb shows excellent performance in all aspects, with a titer of more than 1: 1,280,000, and thus is suitable as an immunodiagnostic reagent for the preparation of an *in vitro* diagnostic kit.

### BRIEF DESCRIPTION OF THE DRAWINGS

FIG. 1 is an electrophoretogram of the MCR-1 protein;
FIG. 2 is an electrophoretogram of the mouse anti-MCR-1 protein antibody; and
FIG. 3 shows a test result of the MCR-1 protein on a test card of the colloidal gold method.

### DETAILED DESCRIPTION OF THE EMBODIMENTS

The embodiments of the present disclosure will be described below.

The present disclosure relates to an antibody 1BH8 produced by a mouse MCR-1 protein-resistant hybridoma cell line and an antibody 1BD9 produced by a mouse MCR-1 protein-resistant hybridoma cell line.

The present disclosure discloses a mouse MCR-1 protein-resistant hybridoma cell line named 1BH8 and a mouse MCR-1 protein-resistant hybridoma cell line named 1BD9.

The antibody 1BH8 produced by a mouse MCR-1 protein-resistant hybridoma cell line includes a light chain variable region and a heavy chain variable region, wherein the light chain variable region includes CDRL1 shown in SEQ ID NO: 1, CDRL2 shown in SEQ ID NO: 2, and CDRL3 shown in SEQ ID NO: 3; and the heavy chain variable region includes CDRH1 shown in SEQ ID NO: 4, CDRH2 shown in SEQ ID NO: 5, and CDRH3 shown in SEQ ID NO: 6; the light chain variable region may have an amino acid sequence shown in SEQ ID NO: 7, and the heavy chain variable region may have an amino acid sequence shown in SEQ ID NO: 9;
SEQ ID NO: 1 RSSQNIVCSYGNPCLE (CDRL1)
SEQ ID NO: 2 KVSNRFS (CDRL2)
SEQ ID NO: 3 FQGSHVPWT (CDRL3)
SEQ ID NO: 4 GYTFTNYIMH (CDRH1)
SEQ ID NO: 5 YINPYNDGTKYNEKFKG (CDRH2)
SEQ ID NO: 6 GPYGNYAMDY (CDRH3)
SEQ ID NO: 7 
DVLMTQTPLSLPVSLGDQASISCRSSQNIVCSYGNPCLEWYLQKPGQSPKLLIYK
VSNRFSGVPDRFSGSGSGTDFTLKISRVEAEDLGVYYCFQGSHVPWTFGGGTELEIKR
SEQ ID NO: 9

VQLQQSGPELVKPGASVKMSCRASGYTFTNYIMHWVKQKPGQGLDWIGYINPY
NDGTKYNEKFKGKATLSSDKSSGTAYMDLSSLTSEDSAVYYCARGPYGNYAMDYW
GQGTSVTVSS

A nucleotide sequence encoding the light chain variable region of the mouse anti-MCR-1 protein antibody 1BH8 may be as shown in SEQ ID NO: 8, and a nucleotide sequence encoding the heavy chain variable region of the antibody 1BH8 may be as shown in SEQ ID NO: 10.
SEQ ID NO: 8 
GATGTTTTGATGACCCAGACGCCACTCTCCCTGCCTGTCAGTCTTGGAGATCA
AGCCTCCATCTCTTGCAGATCTAGTCAGAACATTGTATGTAGTTATGGAAACCCCT
GTTTAGAATGGTACCTGCAGAAACCAGGCCAGTCTCCAAAGCTCCTGATCTACAA
AGTTTCCAACCGATTTTCTGGGGTCCCAGACAGGTTCAGTGGCAGTGGATCAGGG
ACAGATTTCACACTCAAGATCAGCAGAGTGGAGGCTGAGGATCTGGGAGTTTATT
ACTGCTTTCAAGGTTCACATGTTCCGTGGACGTTCGGTGGAGGCACCGAGCTGGA
AATCAAACGG
SEQ ID NO: 10

GTGCAGCTTCAGCAATCAGGACCTGAGCTGGTAAAGCCTGGGGCTTCAGTGA
AGATGTCCTGCAGGGCTTCTGGATACACATTCACTAACTATATTATGCACTGGGT
GAAGCAGAAGCCTGGGCAGGGCCTTGATTGGATTGGATATATTAATCCTTACAAT
GATGGTACTAAGTACAATGAGAAGTTCAAAGGCAAGGCCACACTGTCTTCAGAC
AAATCCTCCGGCACAGCCTATATGGACCTCAGCAGCCTGACCTCTGAGGACTCTG
CGGTCTATTACTGTGCGAGAGGCCCCTATGGTAACTATGCTATGGACTACTGGGG
TCAAGGAACCTCAGTCACCGTCTCTTCA

The antibody 1BD9 produced by a mouse MCR-1 protein-resistant hybridoma cell line includes a light chain variable region and a heavy chain variable region, wherein the light chain variable region includes CDRL1 shown in SEQ ID NO: 11, CDRL2 shown in SEQ ID NO: 12, and CDRL3 shown in SEQ ID NO: 13; and the heavy chain variable region includes CDRH1 shown in SEQ ID NO: 14, CDRH2 shown in SEQ ID NO: 15, and CDRH3 shown in SEQ ID NO: 16, the light chain variable region may have an amino acid sequence shown in SEQ ID NO: 17, and the heavy chain variable region may have an amino acid sequence shown in SEQ ID NO: 19;
SEQ ID NO: 11 RASQDISNYLN (CDRL1)
SEQ ID NO: 12 YTSRLHS (CDRL2)
SEQ ID NO: 13 QQGDMIPDT (CDRL3)
SEQ ID NO: 14 GYAFTSCNMY (CDRH1)
SEQ ID NO: 15 YFDPYSGDAYYNQKFKD (CDRH2)
SEQ ID NO: 16 WLQLYYALDY (CDRH3)
SEQ ID NO: 17 
AIQMTQTPSSLSASLGDRVTISCRASQDISNYLNWYQQKPDGTVKLLIYYTSRLHS
GVPSRFSGSGSGTDYSLNISNLEQEDFATYFCQQGDMIPDTFGGGTKLEIKRADAAPT
SEQ ID NO: 19

VKLQQSGPELVKPGASVKVSCKASGYAFTSCNMYWVKQSHGKSLEWIGYFDPY
SGDAYYNQKFKDKATLTVDKSSSTAYMHLNSLTSEDSAVYYCASWLQLYYALDYW
GQGTSVTVSAAKTT

A nucleotide sequence encoding the light chain variable region of the mouse anti-MCR-1 protein antibody 1BD9 may be as shown in SEQ ID NO: 18, and a nucleotide sequence encoding the heavy chain variable region of the antibody 1BH8 may be as shown in SEQ ID NO: 20.
SEQ ID NO: 18 
GCTATCCAGATGACACAGACTCCATCCTCCCTGTCTGCCTCTCTGGGAGACAG
AGTCACCATCAGTTGCAGGGCAAGTCAGGACATTAGCAATTATTTAAACTGGTAT
CAGCAGAAACCAGATGGAACTGTTAAACTCCTGATCTACTACACATCAAGATTAC
ACTCAGGAGTCCCATCAAGGTTCAGTGGCAGTGGGTCTGGAACAGATTATTCTCT
CAACATTAGCAACCTGGAGCAGGAAGATTTTGCCACTTACTTTTGCCAACAGGGT
GATATGATTCCGGACACGTTCGGAGGGGGGACCAAACTGGAAATAAAACGGGCT
GATGCTGCACCAACT
SEQ ID NO: 20

GTGAAGCTGCAGCAATCTGGACCTGAGCTGGTGAAGCCTGGGGCCTCAGTGA
AGGTCTCCTGCAAGGCTTCTGGTTATGCATTCACTAGCTGCAACATGTACTGGGT
GAAACAGAGCCATGGAAAGAGCCTTGAGTGGATTGGATATTTTGATCCTTACAGT
GGTGATGCTTACTACAACCAGAAGTTCAAGGACAAGGCCACATTGACTGTTGACA
AGTCCTCCAGCACAGCCTACATGCATCTCAACAGCCTGACATCTGAGGACTCTGC

AGTCTATTACTGTGCAAGTTGGTTACAACTTTACTATGCTCTAGACTACTGGGGTC
AAGGAACCTCAGTCACCGTCTCCGCAGCCAAAACGACA

The mouse anti-MCR-1 protein antibodies produced by the hybridoma cell line 1BH8 and the hybridoma cell line 1BD9 can be used as reagents to detect an MCR-1 protein antigen. The mouse anti-MCR-1 protein antibody shows prominent performance in all aspects, and thus is suitable as an immunodiagnostic reagent for the preparation of an *in vitro* diagnostic kit, and the *in vitro* diagnostic kit may be a colloidal gold immunoassay kit, a chemiluminescence kit, a radioimmunoassay kit, an ELISA kit, or a fluoroimmunoassay kit. Alternatively, the mouse anti-MCR-1 protein antibody can be made into a microfluidic chip for detecting an MCR-1 protein antigen.

To develop an MCR-1 protein diagnostic reagent of the colloidal gold method, an MCR-1 protein first needs to be extracted to obtain a high-purity antigen, the antigen is used to stimulate an excellent immune response in mice, and then the hybridoma technique is used to screen an antibody with high affinity and specificity for the development of related *in vitro* diagnostic reagents. The two antibodies involved in this solution are especially suitable for preparing an immune colloidal gold test strip of a double-antibody sandwich method, wherein the antibody 1BH8 is used as a coating antibody and the antibody 1BD9 is used as a gold-labeled antibody. The prepared immune colloidal gold test strip of a double-antibody sandwich method is more sensitive. Moreover, the antibody 1BH8 can be used as a gold-labeled antibody, and the antibody 1BD9 can be used as a coating antibody. The present disclosure is further described below through specific examples. Experimental methods for which operation steps are not specified are all conducted in accordance with the corresponding product instructions. The instruments, reagents, and consumables used in the examples can be purchased from commercial companies unless otherwise specified.

### Example 1: Preparation of a mouse anti-MCR-1 protein antibody

### 1.1 MCR-1 protein antigen preparation

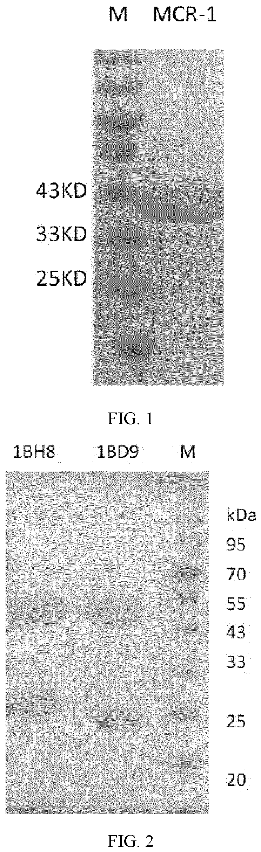

A *mcr-1* gene sequence was searched and downloaded from National Center for Biotechnology Information (NCBI), and a recombinant plasmid was transformed into *Escherichia coli* (*E. coli*) for induced expression. Purification was conducted by nickel-column affinity chromatography. Bacterial cells (with a wet weight of 6 g) were resuspended in an equilibration buffer with pH 7.4 and then subjected to ultrasonication until a bacterial solution was clear, the bacterial solution was centrifuged at a high speed, and a resulting supernatant was filtered through a membrane and then passed through a nickel column to elute a target protein, which had a molecular weight consistent with the expected 40.6 KDa. A sodium dodecyl sulphate-polyacrylamide gel electrophoresis (SDS-PAGE) result was shown in FIG. 1. The protein was quantified by bicinchoninic acid (BCA) assay and then dispensed.

### 1.2 Mouse immunization

6-week-old female Balb/c mice were immunized with the purified MCR-1 protein to prepare an antibody. The mice were divided into 2 groups according to the immunization dosage, with 5 mice in each group; according to an antigen content, a first group was immunized at an immunization dosage of 25 µg/mouse, and a second group was immunized at an immunization dosage of 50 µg/mouse. For an initial immunization, an appropriate amount of the MCR-1 protein was taken and diluted with normal saline to 500 µL, then 500 µL of complete Freund's adjuvant (CFA) was added for thorough emulsification, and a resulting emulsion was subcutaneously injected into the mice (multi-point injection); two weeks later, the mice were subjected to a secondary immunization by intraperitoneal injection at the same dosage, with an adjuvant of incomplete Freund's adjuvant (IFA) instead; two weeks later, the mice were subjected to an additional immunization by intraperitoneal injection; 7 days later, blood was collected from the tail of mice, and a serum titer of the mice was determined by ELISA. Specific steps were as follows: An ELISA plate was coated overnight at 4°C with the MCR-1 protein of 0.2 µg/ml at 100 µL/well, spin-dried, and washed 3 times with phosphate-buffered saline with Tween 20 (PBST). 5% skimmed milk powder was added at 200 µL/well to block at 37°C for 2 h. Blood was collected from the tail of the mice and then centrifuged at 3,000 rpm to obtain serum, and the serum was serially diluted from 1: 1,000 to 1:512,000 with PBS for later use. The plate was spin-dried and then washed 3 times with PBST, a primary antibody diluted with PBS at 1: 1,000 was added at 100 µL/well, and the plate was incubated at 37°C for 1 h. The plate was spin-dried and then washed 3 times with PBST, a goat anti-mouse secondary antibody diluted with PBS at 1:10,000 was added at 100 µL/well, and the plate was incubated at 37°C for 45 min. The plate was spin-dried and then washed 5 times with PBST, TMB was added at 100 µL/well, and the plate was incubated at 37°C to allow a chromogenic reaction for 10 min; the chromogenic reaction was stopped, and a value was read.

### 1.3 Cell fusion

Three days before fusion, mice were subjected to a booster immunization by intraperitoneal injection at the same dosage as the previous immunization, without an adjuvant. Feeder cells were prepared one day before fusion. A Balb/c mouse 6-8 weeks old was selected, and eyeballs of the mouse were removed to collect blood; then the mouse was sacrificed by cervical dislocation, disinfected in 75% alcohol for 5 min, and fixed on a tray; and the abdominal skin was aseptically cut in a clean bench. 10 ml of an HAT selection medium was injected into the abdominal cavity of the mouse with a sterile syringe, the abdomen was slightly rubbed with an alcohol cotton ball, and the medium was drawn out. The medium was added to 40 ml of an HAT medium, and a resulting mixture was added to 4 96-well cell culture plates at 100 µL/well, and then cultivated in a 37°C and 5% CO₂ incubator. One week before fusion, myeloma cells (Sp2/0 cells) were recovered, cultivated in a PRMI-1640 medium with 10% fetal bovine serum (FBS), and subcultivated in a 37°C and 5% CO₂ incubator. Cells in a logarithmic growth phase were collected into a centrifuge tube, subjected to cell counting, and diluted to 10⁷ cells/ml for later use. The Balb/c mice that had been subjected to the booster immunization 3 days ago were taken, eyeballs were removed to collect blood and prepare positive serum, and the mice were sacrificed by cervical dislocation and disinfected with 75% alcohol for 5 min; then a spleen was aseptically collected in a clean bench and washed several times in a sterile dish, and a connective tissue was stripped. The spleen was placed on a microporous copper mesh, a fresh RPMI-1640 medium was added, and the medium was first drawn with a syringe and injected from an end of the spleen to blow off spleen cells; after the process was repeated several times, an inner plug of the syringe was used to gently grind the remaining spleen until there was no obvious red tissue mass. A spleen cell suspension in a dish was gently pipetted up and down, then transferred to a 50 ml centrifuge tube, and centrifuged at 1,000 r/min for 5 min to collect spleen cells, and the spleen cells were counted for later use. The immunized mouse spleen cells and Sp2/0 cells were mixed at a cell number ratio of 10:1, added to a 50 ml centrifuge tube, and centrifuged at 1,000 r/min for 5 min, a resulting supernatant was discarded, and the two cells were thoroughly mixed by rubbing gently in the palm of hands. The centrifuge tube was placed in a 100 mL blue-cap bottle filled with 37°C hot water, and then 1 ml of preheated dimethyl sulfoxide (DMSO)/polyethylene glycol (PEG) was added dropwise to the fusion tube within 1 min, during which the DMSO/PEG was added slowly first and then quickly, and the centrifuge tube was gently swirled. Then an antibiotic-free and serum-free RPMI-1640 medium was immediately added to stop the reaction, during which 1 ml of the medium was added in the first minute, 2 ml of the medium was added in the second minute, 3 ml of the medium was added in the third minute, and 4 ml of the medium was added in the fourth minute. A resulting mixture was incubated in a 37°C water bath for 5 min and then centrifuged at 800 r/min for 5 min, and a resulting supernatant was discarded; a resulting precipitate was suspended with HAT, then thoroughly mixed with 40 ml of an HAT selection medium including 20% calf serum preheated at 37°C, and added to a 96-well cell plate with feeder cells at 100 µL/well, and the cell plate was incubated in a 37°C and 5% CO₂ incubator. 7 days later, half of the medium in the cell plate was replaced with fresh HAT medium, and 10 days later, the medium was completely replaced with HT medium. Positive cells in the 96-well plate were subcloned by limiting dilution analysis (LDA): feeder cells were prepared first according to the above method, and hybridoma cells to be cloned were taken and counted, then diluted to 5 to 8 cells/ml with HT medium, added at 100 µL/well to a 96-well cell plate with the feeder cells (the cloning of each hybridoma cell line required a 96-well cell plate), and cultivated in a 37°C and 5% CO₂ incubator; the number of clones in each cell well was counted and marked about 5 days later, the medium was replaced with fresh medium on day 7, and a test was conducted when cells covered 1/3 to 1/2 of the entire bottom surface of each well; after 2 to 3 times of cloning, all cell wells of the 96-well plate were positive, at which point the cells could be expanded and verified; the obtained two mouse MCR-1 protein-resistant hybridoma cell lines were 1BH8 and 1BD9, respectively, which were cryopreserved. Hybridoma cells verified as positive were expanded and cryopreserved. A specific process was as follows: vigorously-growing hybridoma cells in prominent conditions were gently blown off from a flask with antibiotic-free and serum-free DMEM and then centrifuged at 1,000 r/min for 5 min, and a resulting supernatant was discarded; a cryopreservation solution (with 40% RPMI-1640 medium, 50% FBS, and 10% DMSO) was added to make cells uniformly dispersed, and a resulting cell suspension was then dispensed into cell cryopreservation tubes; then the cryopreservation tubes were placed in a cryopreservation box, the cryopreservation box was placed in a -70°C freezer, and one day later, the cryopreservation tubes were transferred to liquid nitrogen and related records were made.

### 1.4 Ascitic fluid preparation

10 to 12-week-old female Balb/c mice were injected intraperitoneally with sterile liquid paraffin at 0.5 mL/mouse, and 7 days later, the mice were injected intraperitoneally with hybridoma cells in a logarithmic growth phase at 5 × 10⁶ cells/mouse. The mice were observed every day. About 7 to 10 days later, abdomens of the mice obviously bulged, at which point the lower abdominal skin was disinfected with a 75% alcohol cotton ball and then the abdominal cavity was punctured with a 16-gauge needle to collect an ascitic fluid. After an ascitic fluid was regenerated and accumulated, the ascitic fluid was collected once again. The collected ascitic fluid was centrifuged at 3,000 r/min for 10 min, and a resulting clear middle layer was collected, filtered with filter paper, dispensed, and stored at -70°C.

### 1.5 Antibody purification

The ascitic fluid was purified with a Protein-G column. Specific steps were as follows: 2 ml of the ascitic fluid was taken and centrifuged at 10,000 g, and a resulting clear layer was collected and thoroughly mixed with 2 ml of a washing buffer to obtain a sample; 20% ethanol was passed through the column, then the column was equilibrated with 8 mL of a washing liquid, and the sample was passed through the column at a flow rate of 8 S/drop; the sample was repeatedly loaded 3 times, and then the column was washed with 15 mL of a washing buffer at a flow rate of 8 S/drop; after the washing was completed, elution was conducted with 10 mL of an elution buffer, and after the elution was completed, a pH was adjusted to 7.4 with 1 M Tris PH = 9; then concentration was conducted with a concentration column, and a resulting concentrate was subjected to dialysis overnight at 4°C with PBS in a 50 kd dialysis bag.

### Example 2: Identification of the mouse anti-MCR-1 protein antibody

### 2.1 Antibody subtype identification

According to the instructions of the SIGMA kit, the mAb subtype identification was conducted by capture-ELISA. Specific steps were as follows: an mAb subtype identification reagent was diluted at 1:1,000, added to an ELISA plate at 100 µL/well, and incubated at 37°C for 1 h; the plate was washed three times with PBST and pat-dried; the antibody was diluted at 1: 1,000 and added at 100 µL/well, and then the plate was incubated at 37°C for 1 h; the plate was washed three times with PBST and pat-dried; an HRP enzyme-labeled goat anti-mouse IgG secondary antibody was diluted at 1: 10,000 and added at 100 µL/well, and then the plate was incubated at room temperature for 30 min; a chromogenic reaction was conducted for 10 min to 20 min. A subtype reagent added in a well with an OD450 reading significantly higher than that of other wells indicated the subtype of the mAb. The antibody subtype of the antibodies 1BH8 and 1BD9 was IgG1.

### 2.2 Antibody titer determination

The antibody titer was determined after purification was conducted by indirect ELISA. Specific steps were as follows: the MCR-1 protein was diluted to 0.2 µg/mL and added to an ELISA plate at 100 µL/well to coat overnight at 4°C, where an uncoated control was set; the plate was spin-dried and then washed 3 times with PBST; 5% skimmed milk powder was added at 200 µL/well to block at 37°C for 2 h; the plate was spin-dried and then washed 3 times with PBST; the antibody (with a concentration of 1 mg/ml) was serially diluted from 1: 1,000 to obtain a total of 12 gradient concentrations, resulting antibody dilutions were each added to the plate at 100 µL/well, and the plate was incubated at 37°C for 1 h, where an uncoated control was set; the plate was spin-dried and then washed 3 times with PBST, a goat anti-mouse secondary antibody diluted with PBS at 1:10,000 was added at 100 µL/well, and the plate was incubated at 37°C for 45 min; the plate was spin-dried and then washed 5 times with PBST, TMB was added at 100 µL/well, and the plate was incubated at 37°C to allow a chromogenic reaction for 10 min; the chromogenic reaction was stopped, and a value was read. After the purification, when the antibody was diluted to 1 mg/ml, a titer reached more than 1: 1,280,000.

### 2.3 Identification of antibody purity and molecular weight

SDS-PAGE was used to identify the molecular weight and purity of the antibody. Gels were prepared, where a separation gel was 12% and a stacking gel was 5%; 20 µL of a sample and 20 µL of a buffer were thoroughly mixed and boiled for 3 min to obtain a sample solution; 20 µL of the sample solution was loaded in each well, and a prestained protein Marker control was set; electrophoresis was conducted at 80 V for 30 min and at 120 V for 2 h; after the electrophoresis was completed, a Coomassie brilliant blue (CBB) solution was added for staining; destaining was conducted by boiling in deionized water 3 times, with 5 min each time; a purified mAb was identified by SDS-PAGE, and resulting bands were clear, without impurity bands. As shown in FIG. 2, there were clear bands at 50 KDa and 25 KDa.

### Example 3: Gene verification of the mouse anti-MCR-1 protein antibody

An Ig variable region gene was cloned by RT-PCR. Total RNA was extracted from the hybridoma cell lines 1BD9 and 1BH8 by the Trizol method (purchased from Invitrogen), and was reverse-transcribed into a cDNA library with M-MLV reverse transcriptase (purchased from Invitrogen).

Upstream primer for a heavy chain framework region
P1: 5'SAGGTGMAGCTKCASSARTCWGG3', as shown in SEQ ID NO: 21
Downstream primer for a heavy chain variable region
P2: 5'TGGGGSTGTYGTTTTGGCTGMRGAGACRGTGA3', as shown in SEQ ID NO: 22
Upstream primer for a light chain leader peptide
P3: 5'ATGGATTTTCAAGTGCAGATTTTCAG3', as shown in SEQ ID NO: 23
Downstream primer for a light chain variable region
P4: 5'GGATACAGTTGGTGCAGCATCAGCCCGTTT3', as shown in SEQ ID NO: 24
A PCR system (50 µL) was prepared as follows:
   cDNA: 2 µL; upstream primer (10 µM): 2 µL; downstream primer (10 µM): 2 µL; dNTP mixture: 2 µL; pfu DNA polymerase (5 U/µL): 1 µL; 10 X pfu Buffer II: 5 µL; and ddHzO: making up to 50 µL.

PCR conditions were as follows: pre-denaturation at 95°C for 5 min; repeating the following cycle 35 times: 95°C for 30 s, 58°C for 30 s, and 72°C for 1 min; and finally, extension at 72°C for 10 min.

VL and VH fragments were separated and recovered by agarose gel electrophoresis. The recovered VL and VH fragments were ligated with a pMD19-T (sKPCle) vector (Takara), and a ligation system was as follows:
VL PCR product/VH PCR product: each 70 ng; pMD19-T (sKPCle) vector: 1 µL; Solution I ligation reaction solution: 5 µL; and ddH₂O: making up to 10 µL. The ligation was conducted overnight at 4°C.

A ligation product was transformed into *E. coli* DH5α competent cells, the competent cells were cultivated overnight at 37°C, single colonies were picked and cultivated at 37°C under shaking for 2 h, and then a resulting bacterial solution was subjected to PCR identification. cDNA of a corresponding antibody was used as a positive control. A reaction system (25 µL) was prepared as follows:
bacterial solution: 1 µL; upstream primer (10 µM): 1 µL; downstream primer (10 µM): 1 µL; dNTP Mixture (each 2.5 Mm): 2 µL; Taq DNA polymerase (5 U/µL): 0.5 µL; 10 × Taq Buffer (Mg²⁺ plus): 2.5 µL; and water: making up to 25 µL. Reaction conditions were the same as above.

PCR-positive clones were selected for expansion, and a plasmid was extracted from the positive clones with a plasmid extraction kit (Takara) and sequenced. At least 5 clone samples were sequenced for each chain of each antibody until at least three samples had the same sequencing result. The heavy chain and light chain variable region sequences of the antibodies 1BD9 and 1BH8 were successfully cloned, which were consistent with the characteristics of typical antibody variable region sequences according to alignment.

### Example 4: Preparation of an MCR-1 protein test card by a colloidal gold method

An immune colloidal gold test strip of a double-antibody sandwich method was prepared as follows:
step 1: a 0.1 M K₂CO₃ solution was added to a colloidal gold solution under stirring, a pH was adjusted, and the mouse anti-MCR-1 mAb 1BD9 was added; a resulting mixture was stirred, a 10% bovine serum albumin (BSA) solution and 2% PEG 20000 were added, and a resulting mixture was stirred and centrifuged at a low speed to obtain a supernatant; then the supernatant was centrifuged at a high speed to obtain a precipitate, and the precipitate was resuspended with colloidal gold to form a gold-labeled antibody;
step 2: the gold-labeled antibody was sprayed on a glass fiber membrane, and then the glass fiber membrane was oven-dried to form a gold-labeled pad;
step 3: a 1% thimerosal sodium solution was thoroughly mixed with the mouse anti-MCR-1 protein mAb 1BH8 to form a test line coating solution; then PBS and a 1% thimerosal sodium solution were added to the goat anti-mouse IgG, and a resulting mixture was thoroughly mixed to form a control line coating solution; the control line coating solution and the test line coating solution were streaked on a nitrocellulose (NC) membrane, and the NC membrane was oven-dried to obtain a coated membrane; and
step 4: the coated membrane was attached to a backing card, the gold-labeled pad and absorbent paper were lapped on the coated membrane, and a resulting product was laminated and cut to obtain the MCR-1 protein test card of the colloidal gold method.

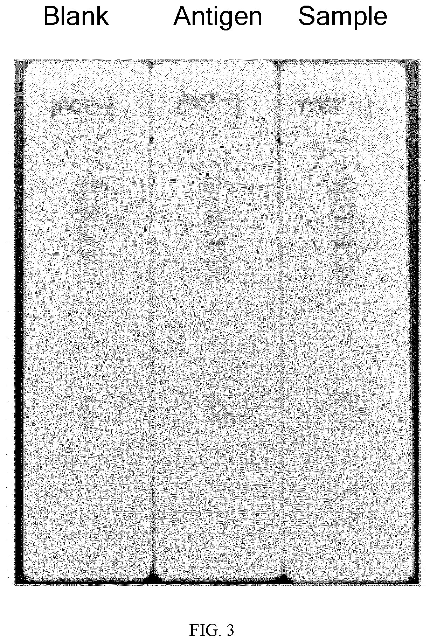

The MCR-1 protein test card of the colloidal gold method prepared by the above method was taken, and a blank sample, an MCR-1 protein, and an MCR-1 protein-containing positive sample were each spotted on the test card. Results were shown in FIG. 3, where test results of the blank sample, the MCR-1 protein, and the MCR-1 protein-containing positive sample were shown from left to right, respectively. It can be seen that the test result of the blank sample was negative, and the test results of the MCR-1 protein and the MCR-1 protein-containing positive sample were all positive, indicating that the MCR-1 protein test card of the colloidal gold method prepared by this solution can detect an MCR-1 protein and a corresponding positive sample.

The examples of the present disclosure are described above in detail, which are merely preferred examples of the present disclosure and cannot be construed as limiting the scope of implementation of the present disclosure.

## Claims

1. A mouse anti-MCR-1 protein antibody, wherein the mouse anti-MCR-1 protein antibody is: an antibody 1BH8 comprising a light chain variable region and a heavy chain variable region, wherein the light chain variable region comprises complementary-determining region (CDR) L1 shown in SEQ ID NO: 1, CDRL2 shown in SEQ ID NO: 2, and CDRL3 shown in SEQ ID NO: 3; and the heavy chain variable region comprises CDRH1 shown in SEQ ID NO: 4, CDRH2 shown in SEQ ID NO: 5, and CDRH3 shown in SEQ ID NO: 6, the light chain variable region has an amino acid sequence shown in SEQ ID NO: 7, and the heavy chain variable region has an amino acid sequence shown in SEQ ID NO: 9;
or,
an antibody 1BD9 comprising a light chain variable region and a heavy chain variable region, wherein the light chain variable region comprises CDRL1 shown in SEQ ID NO: 11, CDRL2 shown in SEQ ID NO: 12, and CDRL3 shown in SEQ ID NO: 13; and the heavy chain variable region comprises CDRH1 shown in SEQ ID NO: 14, CDRH2 shown in SEQ ID NO: 15, and CDRH3 shown in SEQ ID NO: 16, the light chain variable region has an amino acid sequence shown in SEQ ID NO: 17, and the heavy chain variable region has an amino acid sequence shown in SEQ ID NO: 19.

2. A nucleic acid, comprising nucleotides encoding the mouse anti-MCR-1 protein antibody according to claim 1.

3. The nucleic acid according to claim 2, wherein a nucleotide sequence of the nucleic acid for encoding the light chain variable region of the antibody 1BH8 is shown in SEQ ID NO: 8, and a nucleotide sequence of the nucleic acid for encoding the heavy chain variable region of the antibody 1BH8 is shown in SEQ ID NO: 10;
or,
a nucleotide sequence of the nucleic acid for encoding the light chain variable region of the antibody 1BD9 is shown in SEQ ID NO: 18, and a nucleotide sequence of the nucleic acid for encoding the heavy chain variable region of the antibody 1BD9 is shown in SEQ ID NO: 20.

4. A reagent for detecting an MCR-1 protein antigen comprising the mouse anti-MCR-1 protein antibody according to claim 1.

5. The reagent according to claim 4, wherein the mouse anti-MCR-1 protein antibody is used in an *in vitro* diagnostic kit or a microfluidic chip; and the *in vitro* diagnostic kit is a colloidal gold immunoassay kit, a chemiluminescence kit, a radioimmunoassay kit, an enzyme-linked immunosorbent assay (ELISA) kit, or a fluoroimmunoassay kit.

6. The reagent according to claim 4, wherein an immune colloidal gold test strip of a double-antibody sandwich method is prepared, wherein the antibody 1BH8 is used as a coating antibody and the antibody 1BD9 is used as a gold-labeled antibody; or
the antibody 1BD9 is used as a coating antibody and the antibody 1BH8 is used as a gold-labeled antibody.

## Patentansprüche

1. Ein Maus-Anti-MCR-1-Protein-Antikörper, wobei der Maus-Anti-MCR-1-Protein-Antikörper ein Antikörper 1BH8 ist, der eine variable Region der leichten Kette und eine variable Region der schweren Kette umfasst, wobei die variable Region der leichten Kette die komplementärbestimmende Region (CDR) L1, die gezeigt ist in SEQ ID NO: 1, CDRL2, die gezeigt in SEQ ID NO: 2, und CDRL3, die gezeigt ist in SEQ ID NO: 3; die variable Region der schweren Kette umfasst CDRH1, gezeigt in SEQ ID NO: 4, CDRH2, gezeigt in SEQ ID NO: 5, und CDRH3, gezeigt in SEQ ID NO: 6, die variable Region der leichten Kette weist eine Aminosäuresequenz auf, die gezeigt ist in SEQ ID NO: 7, und die variable Region der schweren Kette eine Aminosäuresequenz aufweist, die gezeigt ist in SEQ ID NO: 9;
oder
einen Antikörper 1BD9, der eine variable Region der leichten Kette und eine variable Region der schweren Kette umfasst, wobei die variable Region der leichten Kette CDRL1, die gezeigt ist in SEQ ID NO: 11, CDRL2, gezeigt in SEQ ID NO: 12, und CDRL3, gezeigt in SEQ ID NO: 13; die variable Region der schweren Kette umfasst CDRH1, gezeigt in SEQ ID NO: 14, CDRH2, gezeigt in SEQ ID NO: 15, und CDRH3, gezeigt in SEQ ID NO: 16, die variable Region der leichten Kette weist eine Aminosäuresequenz auf, die gezeigt ist in SEQ ID NO: 17, und die variable Region der schweren Kette eine Aminosäuresequenz aufweist, die gezeigt ist in SEQ ID NO: 19.

2. Eine Nukleinsäure, umfassend Nukleotide, die den Maus-Anti-MCR-1-Protein-Antikörper nach Anspruch 1 kodieren.

3. Nukleinsäure nach Anspruch 2, wobei eine Nukleotidsequenz der Nukleinsäure zum Kodieren der variablen Region der leichten Kette des Antikörpers 1BH8 die gezeigt ist in SEQ ID NO: 8, und eine Nukleotidsequenz der Nukleinsäure zum Kodieren der variablen Region der schweren Kette des Antikörpers 1BH8, die gezeigt ist in SEQ ID NO: 10;
oder
eine Nukleotidsequenz der Nukleinsäure zum Kodieren der variablen Region der leichten Kette des Antikörpers 1BD9, die gezeigt ist in SEQ ID NO: 18, und eine Nukleotidsequenz der Nukleinsäure zum Kodieren der variablen Region der schweren Kette des Antikörpers 1BD9, die gezeigt ist in SEQ ID NO: 20.

4. Ein Reagenz zum Nachweis eines MCR-1-Protein-Antigens, das den Maus-Anti-MCR-1-Protein-Antikörper gemäß Anspruch 1 umfasst.

5. Das Reagenz nach Anspruch 4, wobei der Maus-Anti-MCR-1-Protein-Antikörper in einem In-vitro-Diagnostik-Kit oder einem mikrofluidischen Chip verwendet wird; und das *In-*vitro-Diagnostik-Kit ein Kolloidalgold-Immunoassay-Kit, ein Chemolumineszenz-Kit, ein Radioimmunoassay-Kit, ein Enzyme-Linked-Immunosorbent-Assay (ELISA)-Kit oder ein Fluorimmunoassay-Kit ist.

6. Das Reagenz nach Anspruch 4, wobei ein Immun-Kolloidgold-Teststreifen eines Doppel-Antikörper-Sandwich-Verfahrens hergestellt wird, wobei der Antikörper 1BH8 als Beschichtungs-Antikörper und der Antikörper 1BD9 als goldmarkierter Antikörper verwendet wird; oder
der Antikörper 1BD9 als Beschichtungs-Antikörper und der Antikörper 1BH8 als goldmarkierter Antikörper verwendet wird.

## Revendications

1. Anticorps anti-protéine MCR-1 de souris, dans lequel l'anticorps anti-protéine MCR-1 est : un anticorps 1BH8 comprenant une région variable de chaîne légère et une région variable de chaîne lourde, dans lequel la région variable de chaîne légère comprend la région de détermination complémentaire (CDR) L1 représentée dans SEQ ID NO : 1, CDRL2 représentée dans SEQ ID NO : 2, et CDRL3 représentée dans SEQ ID NO : 3 ; et la région variable de chaîne lourde comprend la CDRH1 représentée dans SEQ ID NO : 4, CDRH2 représentée dans SEQ ID NO : 5, et CDRH3 représentée dans SEQ ID NO : 6, la région variable de chaîne légère a une séquence d'acides aminés représentée dans SEQ ID NO : 7, et la région variable de chaîne lourde a une séquence d'acides aminés représentée dans SEQ ID NO : 9 ;
ou,
un anticorps 1BD9 comprenant une région variable de chaîne légère et une région variable de chaîne lourde, dans lequel la région variable de chaîne légère comprend CDRL1 représentée dans SEQ ID NO : 11, CDRL2 représentée dans SEQ ID NO : 12, et CDRL3 représentée dans SEQ ID NO : 13 ; et la région variable de chaîne lourde comprend la CDRH1 représentée dans SEQ ID NO : 14, CDRH2 représentée dans SEQ ID NO : 15, et CDRH3 représentée dans SEQ ID NO : 16, la région variable de chaîne légère a une séquence d'acides aminés représentée dans SEQ ID NO : 17, et la région variable de chaîne lourde a une séquence d'acides aminés représentée dans SEQ ID NO : 19.

2. Acide nucléique, comprenant des nucléotides codant pour l'anticorps anti-protéine MCR-1 de souris selon la revendication 1.

3. Acide nucléique selon la revendication 2, dans lequel une séquence nucléotidique de l'acide nucléique pour coder la région variable de chaîne légère de l'anticorps 1BH8 est représentée dans SEQ ID NO : 8, et une séquence nucléotidique de l'acide nucléique pour coder la région variable de chaîne lourde de l'anticorps 1BH8 est représentée dans SEQ ID NO : 10 ;
ou,
une séquence nucléotidique de l'acide nucléique pour coder la région variable de chaîne légère de l'anticorps 1BD9 est représentée dans SEQ ID NO : 18, et une séquence nucléotidique de l'acide nucléique pour coder la région variable de chaîne lourde de l'anticorps 1BD9 est représentée dans SEQ ID NO : 20.

4. Réactif pour la détection d'un antigène de protéine MCR-1 comprenant l'anticorps anti-protéine MCR-1 de souris selon la revendication 1.

5. Réactif selon la revendication 4, dans lequel l'anticorps anti-protéine MCR-1 de souris est utilisé dans un kit de diagnostic *in vitro* ou une puce microfluidique ; et le kit de diagnostic *in vitro* est un kit de dosage immunologique d'or colloïdal, un kit de chimioluminescence, un kit de dosage radio-immunologique, un kit de dosage d'immunoabsorption enzymatique (ELISA), ou un kit de dosage immunologique au fluor.

6. Réactif selon la revendication 4, dans lequel une bande de test d'or colloïdal immunitaire d'un procédé sandwich à double anticorps est préparée, dans lequel l'anticorps 1BH8 est utilisé en tant qu'anticorps de revêtement et l'anticorps 1BD9 est utilisé en tant qu'anticorps marqué à l'or ; ou
l'anticorps 1BD9 est utilisé en tant qu'anticorps de revêtement et l'anticorps 1BH8 est utilisé en tant qu'anticorps marqué à l'or.
